# EUROPÄISCHE PATENTANMELDUNG

(11) **EP 0 834 545 A2**
(43) Veröffentlichungstag der Anmeldung: **08.04.1998**
(21) Anmeldenummer: 97116514.7
(22) Anmeldetag: 23.09.1997
(51) Int. Cl.: C11D 1/12, C07C 303/22

(54) **Tensidmischungen mit einem Gehalt an Acyloxialkansulfonaten**

(30) Priorität: 01.10.1996 DE 19640572
(71) Anmelder: Clariant GmbH, 65929 Frankfurt am Main (DE)
(72) Erfinder: Nestler, Bernd, Dr., 65929 Frankfurt (DE)

(57) **Zusammenfassung**

Tensidmischungen auf der Basis von Acyloxialkansulfonaten, hergestellt durch Umsetzung von mehr als einem Mol einer oder mehrerer Fettsäuren mit einem Mol eines Gemisches aus Alkali- und/oder Erdalkali-hydroxialkansulfonat und Ammonium-hydroxialkansulfonat in Gegenwart eines Veresterungskatalysators bei einer Temperatur von 100 bis 260°C.

## Beschreibung

Acyloxialkansulfonate stellen anionische Tenside dar, die als Rohstoffe für Syndet-Seifen, kosmetische Mittel und Reinigungsformulierungen Verwendung finden. Sie zeichnen sich durch gute Schaumeigenschaften, gute Hartwasserstabilität und gute Hautverträglichkeit aus.

Nachteilig für die Verwendung dieser Tenside ist, daß es sich dabei meist um spröde Feststoffe handelt, die erst bei hohen Temperaturen schmelzen bzw. rührbar sind. Bei diesen hohen Temperaturen, die erforderlich sind, um das Acyloxialkansulfonat überhaupt erst verarbeitbar machen, ist dieses sehr oxidationsempfindlich, eine thermische Zersetzung setzt ein und Verfärbungen treten auf.

Demnach ist es vorteilhaft den Schmelzpunkt bzw. die Temperatur, bei der Acyloxialkansulfonate rührbar und somit verarbeitbar sind, zu senken. Zur Lösung dieses Problems ist es bereits bekannt, solche Acyloxialkansulfonate herzustellen, die gemischte Salze darstellen, wo also das Kation aus einem Gemisch von zwei unterschiedlichen Kationen, beispielsweise Natrium- und Kalium-Ion besteht (US 3 029 264). Diese Acyloxialkansulfonate, wobei es sich vorzugsweise um Acyloxiisethionate handelt, werden durch Veresterung von Fettsäuren mit einem Salz der Isethionsäure hergestellt. Nimmt man nun entsprechend den Angaben des genannten Standes der Technik ein Gemisch von Na-Isethionat und K-Isethionat, so kann man die Veresterung mit der Fettsäure bereits bei 150 bis 160°C durchführen. Auf diese Weise läßt sich die Bildung von Verfärbungen vermeiden. Bei diesem Verfahren werden jedoch in allen Fällen äquimolare Mengen an Fettsäure und Isethionat umgesetzt. Es wurde nun gefunden, daß man bei solchen Acyloxialkansulfonaten mit gemischten Kationen die Rührbarkeitsgrenze, d.h. die Temperatur, bei der das Produkt noch gerührt werden kann, noch herabsetzen kann, wenn man anstelle äquimolarer Mengen einen Überschuß an Fettsäure nimmt.

Acyloxialkansulfonate mit gemischten Kationen sind auch in WO 94/09107 beschrieben. Dort fehlt jedoch jeder Hinweis auf die Auswirkungen der gemischten Kationen auf die Herstellbedingungen.

Gegenstand der Erfindung sind Tensidmischungen auf der Basis von Acyloxialkansulfonaten, hergestellt durch Umsetzung von mehr als einem Mol einer oder mehrerer Fettsäuren mit einem Mol eines Gemisches aus Alkali- und/oder Erdalkali-hydroxialkansulfonat und Ammonium-hydroxialkansulfonat in Gegenwart eines Veresterungskatalysators bei einer Temperatur von 150 bis 200°C.

Als Fettsäuren kommen gesättigte oder ungesättigte Fettsäuren in Frage mit einem Gehalt von 8 bis 32 C-Atomen. Als Beispiele seien genannt Capronsäure, Caprinsäure, Laurinsäure, Myristinsäure, Stearinsäure, Arachinsäure, Ölsäure, Linolsäure, Linolensäure. Bevorzugt sind Mischungen von Fettsäuren, wie beispielsweise Cocosfettsäure und Talgfettsäure. Neben unverzweigten sind auch verzweigte Fettsäuren geeignet, beispielsweise 2-Ethylhexansäure, 2-Pentyloctansäure, 2-Butylnonansäure, 2-Propyldecansäure, 2-Ethylundecansäure, 2-Butylundecansäure, 2-Methyldodecansäure, 2-Ethyltridecansäure und 2-Methyltetradecansäure und Mischungen davon.

Die Hydroxyalkansulfonate entsprechen der Formel HO-R¹-SO₃X, worin R¹ -CH₂CH₂-, -(CH₂)₃-, -CH₂CH(CH₃)- oder -CH₂CH₂OCH₂CH₂- ist, wobei Ethylen bevorzugt ist.
X bedeutet hierin eine äquivalente Menge einer Mischung von Alkali- und/oder Erdalkali-kationen, vorzugsweise Natrium oder Kalium und Ammonium-kationen. Die Ammonium-kationen haben die allgemeine Formel 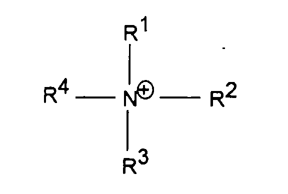 worin R¹, R², R³ und R⁴ gleich oder verschieden sein können und Wasserstoff oder C₁-C₄-Alkyl, vorzugsweise Methyl oder Ethyl oder C₁-C₄-Hydroxyalkyl, bedeuten, wobei R¹ bis R⁴ gleich oder verschieden sein können. Bevorzugt sind Natrium-, Kalium-, ⁺NH(CH₃)₃, ^{⊕}NH(C₂H₅)₃, HN^{⊕}(CH₂CH₂OH)₃ und NH^{⊕}₄-Kationen. Das Mischungsverhältnis der einzelnen Kationen kann innerhalb weiter Grenzen schwanken, wichtig ist in jedem Fall, daß X in der obigen Formel nicht ein einzelnes Kation, sondern eine Mischung verschiedener Kationen darstellt. Ein Teil dieser Mischung der verschiedenen Kationen ist in jedem Fall ein beliebiges Ammonium-kation der zuvor genannten Formel, der Rest ist dann beispielsweise ein Natrium- oder Kalium-Ion. Möglich sind auch Mischungen von drei oder mehreren verschiedenen Kationen, beispielsweise Mischungen von Natrium-, Kalium- und Ammonium-Ionen.

Bevorzugt sind erfindungsgemäße Tensidmischungen, die so hergestellt werden, daß darin die Acyloxialkansulfonate mit folgenden molaren Verhältnissen der Kationen vorliegen:
K:NH₄ von 97:3 bis 5:95; Na:NH₄ von 98:2 bis 5:95, insbesondere 97:3 bis 50:50; Na:NH(C₂H₅)₃ von 98:2 bis 10:90; K:NH(C₂H₅)₃ von 98:2 bis 10:90.

Die erfindungsgemäßen Tensidmischungen werden bevorzugt nach dem Verfahren der sogenannten Direktveresterung durch Reaktion eines Überschusses von Fettsäure mit dem Hydroxialkansulfonat in Gegenwart eines Veresterungskatalysators bei einer Temperatur von 100 bis 260°C unter gleichzeitiger Entfernung von vorhandenem Wasser hergestellt. Diese Direktveresterung erfolgt im einzelnen analog zu den Angaben in EP-A-0 585 071 (US 5,384,421), die hier miteinbezogen wird.

Wesentlich für die erfindungsgemäßen Tensidmischungen ist, daß man zu deren Herstellung einen molaren Überschuß an Fettsäure, bezogen auf Hydroxialkansulfonat, nimmt. Bevorzugt ist ein Überschuß von bis zu 2 Mol Fettsäure auf 1 Mol Hydroxialkansulfonsäure.

Die Hydroxyalkansulfonsäuresalze können als solche eingesetzt werden, bevorzugt werden sie in Form einer wäßrigen Lösung, im allgemeinen als 40 bis 65 gew.-%ige Lösung, eingesetzt.

Geeignete Veresterungskatalysatoren sind in der genannten EP-A-0 585 071 ausführlich beschrieben, die hier miteinbezogen wird. Es handelt sich um Alkansulfonsäuren, Hydroxyalkansulfonsäuren, Arylsulfonsäuren, anorganische Säuren wie Schwefelsäure, Phosphorsäure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, bezogen auf Hydroxyalkansulfonat.

Die Veresterung kann im einzelnen in der Weise durchgeführt werden, daß man bei Atmosphärendruck die Fettsäure, das Hydroxyalkansulfonat und den Veresterungskatalysator in einem Reaktionsgefäß vorlegt und die Mischung unter Rühren auf die oben angegebene Temperatur erhitzt. Das gegebenenfalls mit den Ausgangskomponenten in die Reaktionsmischung eingebrachte Wasser und das durch die Veresterungsreaktion entstehende Wasser wird dabei kontinuierlich aus der Reaktionsmischung ausgetragen. Darüberhinaus kann es auch sinnvoll sein, im Verlauf der Veresterungsreaktion einen Teil der überschüssigen Fettsäure abzudestillieren.

Man kann die Veresterungsreaktion auch anfangs bei Atmosphärendruck und dann unter Anwendung eines Vakuums zur schnelleren Austragung des Wassers durchführen. Die Zeit bis zum angestrebten Umsatz von Fettsäure oder von Ammonium-Hydroxyalkansulfonat liegt bei etwa 4 bis 8 Stunden. Im allgemeinen wird man, zum Beispiel aus Zeitgründen, einen 100%igen Umsatz nicht anstreben, sondern bei einem niedrigeren Prozentsatz, zum Beispiel bei 75 bis 90 Gew.-% Acyloxyalkansulfonat, die Veresterungsreaktion abbrechen, zum Beispiel durch Abkühlen. Das erhaltene Reaktionsprodukt ist bei Raumtemperatur flüssig oder fest. Eine Konfektionierung des bei Raumtemperatur festen Produkts kann man zum Beispiel mit Hilfe einer Schuppenwalze oder eines Kühlbandes durchführen.

Um die Viskosität des Reaktionsgemisches zu vermindern, kann man dem Reaktionsgemisch vor oder während des Abkühlvorgangs sogenannte Konsistenzregler zugeben. Hierfür kommen in Frage beispielsweise Paraffine, wie in EP-A-0 585 071 beschrieben, Fettsäuren, Fettsäureester, Polyethylenglykole oder Mischungen von Konsistenzreglern. Bevorzugt sind freie Fettsäuren, und zwar solche, die eine andere Kettenlänge haben als die zur Herstellung des Acyloxialkansulfonats benutzte Fettsäure. Der Anteil dieser Konsistenzregler kann bis zu 60 Gew.-%, vorzugsweise bis zu 30 Gew.-% betragen. Bevorzugt sind Mischungen mit bis zu 30 Gew.-% Paraffin, bis zu 50 Gew.-% Fettsäure und bis zu 10 Gew.-% Polyethylenglykol. Die Prozentangaben beziehen sich jeweils auf den Gehalt an das das Acyloxialkansulfonat enthaltende Material.

Die auf die beschriebene Art und Weise hergestellten Tensidmischungen, die als Hauptkomponente Acyloxialkansulfonate mit gemischten Kationen und daneben Restmengen an freien Fettsäuren und gegebenenfalls Konsistenzregler enthalten, zeichnen sich gegenüber ähnlichen Produkten nach dem Stand der Technik (US 3,029,264) dadurch aus, daß bei gleichem Gehalt an Acyloxialkansulfonat die Temperaturgrenze, bei der das Tensidgemisch noch rührbar ist, deutlich niedriger ist. Diese Absenkung der Rührbarkeitsgrenze bei den erfindungsgemäßen Tensidmischungen hat zur Folge, daß hier der Gehalt an Acyloxialkansulfonat höher sein kann als bei den Mischungen des genannten Standes der Technik. Eine weitere Absenkung der Rührbarkeitsgrenze kann durch Zugabe von Konsistenzreglern erfolgen. Darüberhinaus ist bei den erfindungsgemäßen Tensidmischungen der Restgehalt an freiem Isethionat deutlich niedriger.

### Beispiele 1 bis 7

In einem 2 l Planschliffbecher mit Rührer, absteigender Destillationsbrücke, Innenthermometer und Stickstoffeinleitung wurden 230 g Cocosfettsäure (1,12 mol), die einer Gesamtmenge von 1.0 mol Isethionat entsprechenden Mengen wäßriger Na- und NH₄-Isethionat-Lösungen und 0.71 g Zinkoxid vorgelegt. Der Ansatz wurde auf 220°C erwärmt und das bei der Direktkondensation gebildete Wasser abdestilliert. Bei einem Gehalt an Acylisethionat (waschaktiver Substanz - WAS) von ca. 80 % (Epton-Titration) wurde das Reaktionsgemisch langsam unter Rühren abgekühlt, und die Rührbarkeitsgrenze, d.h. die Temperatur bei der das Gemisch noch rührbar ist, der WAS-Gehalt, sowie der Gehalt an freiem Hydroxyethansulfonat-Anion bestimmt. Folgende Resultate wurden erhalten:

| Bsp. Nr. | Verhältnis Na/NH₄-Isethionat | | rührbar bis [°C] | WAS [%] | Hydroxyethansulfonat-Anion [Gew.-%] |
|---|---|---|---|---|---|
| | Na | NH₄ | | | |
| 1 | 100 | 0 | 195 | 81 | 4.2 |
| 2 | 95 | 5 | 95 | 80 | 4.3 |
| 3 | 90 | 10 | 105 | 79 | 4.3 |
| 4 | 85 | 15 | 170 | 80 | 4.1 |
| 5 | 70 | 30 | 180 | 80 | 3.5 |
| 6 | 30 | 70 | 175 | 79 | 2.2 |
| 7 | 0 | 100 | 180 | 81 | 3.3 |

### Beispiele 8 bis 14

Entsprechend den vorherigen Beispielen wurden Acylisethionat-Schmelzen hergestellt, in die jeweils zusätzlich 62 g Stearinsäure als Konsistenzregler eingerührt wurden. Anschließend wurde abgekühlt und die Rührbarkeitsgrenze, der WAS-Gehalt, sowie der Gehalt an freiem Hydroxyethansulfonat-Anion des Endprodukts ermittelt. Eine Zusammenfassung der Ergebnisse gibt folgende Tabelle:

| Bsp. Nr. | Verhältnis Na/NH₄-Isethionat | | rührbar bis [°C] | WAS [%] | Hydroxyethansulfonat-Anion [Gew.-%] |
|---|---|---|---|---|---|
| | Na | NH₄ | | | |
| 8 | 100 | 0 | 150 | 67 | 3.6 |
| 9 | 95 | 5 | 78 | 73 | 3.7 |
| 10 | 90 | 10 | 70 | 69 | 3.7 |
| 11 | 85 | 15 | 92 | 68 | 3.5 |
| 12 | 70 | 30 | 105 | 68 | 3.0 |
| 13 | 30 | 70 | 110 | 68 | 1.9 |
| 14 | 0 | 100 | 140 | 69 | 2.8 |

### Beispiel 15

Entsprechend den Beispielen 6 - 12 wurden 224 g Laurinsäure, 76,6 g wäßrige Natriumisethionat-Lösung (58 %), 181 g wäßrige Ammoniumisethionat-Lösung (55 %) und 0,71 g Zinkoxid vorgelegt und der Ansatz bei 180°C kondensiert. Bei einem WAS-Gehalt von 81 % (Epton-Titration) war das Gemisch nicht mehr rührbar, der Gehalt an freiem Hydroxyethansulfonat-Anion lag bei 2,2 %.

Durch Zusatz von 62 g Stearinsäure konnte die Rührbarkeitsgrenze auf 130°C abgesenkt werden, das resultierende produkt wies einen Gehalt an waschaktiver Substanz von 70 % (Epton-Titration) und einen Gehalt an freiem Hydroxyethansulfonat von 1,9 % auf.

### Beispiel 16 bis 18

In einem 3 l Planschliffbecher mit Rührer, absteigender Destillationsbrücke, Innenthermometer und Stickstoffeinleitung wurden 283 g Cocosfettsäure, 243 g wäßrige Natriumisethionat-Lösung (58 %) und 13,5 g wäßrige Ammoniumisethionat-Lösung (53 5) vorgelegt und der Ansatz bei 180°C unter Abdestillieren des bei der Reaktion gebildeten Wassers bis zu einem Gehalt an waschaktiver Substanz von 77 % kondensiert. Nach Zusatz geeigneter Additive (siehe Tabelle) wurden die WAS-Gehalte und Rührbarkeitsgrenzen der derart erhaltenen Natrium-/Ammoniumcocoylisethionat-Schmelzen bestimmt.

| Versuch Nr. | Additive | rührbar bis [°C] | WAS [%] |
|---|---|---|---|
| 16 | 158 g Paraffin | 60 | 56 |
| 17 | 158 g Stearinsäure | 65 | 56 |
| 18 | 48.3 Paraffin | 45 | 56 |
| | 58.5 g Stearinsäure | | |
| | 25.6 g Laurinsäure | | |
| | 25.6 g Polyethylenglykol | | |

### Beispiel 19

In einem Planschliffbecher mit Rührer, absteigener Destillationsbrücke, Innenthermometer und Stickstoffeinleitung wurden 308 g Cocosfettsäure, 253 g wäßrige Natriumisethionat-Lösung (58 %), 141 g wäßrige Kaliumisethionat-Lösung (53 %) und 16.2 g wäßrige Ammoniumisethionat-Lösung (53 %) und 1.1 g Zinkoxid vorgelegt. Der Ansatz wurde auf 220°C erwärmt und das bei der Direktkondensation gebildete Wasser abdestilliert. Bei einem Gehalt an waschaktiver Substanz (WAS) von 84 % (Epton-Titration) wurde das Reaktionsgemisch langsam unter Rühren abgekühlt. Das derart erhaltene Natrium-/Kalium-Ammoniumcocoylisethionat-Gemisch war bis zu einer Temperatur von 145°C rührbar.

### Beispiel 20

Entsprechend dem vorherigen Beispiel wurde eine Natrium-/Kalium-/Ammoniumcocoylisethionat-Schmelze hergestellt. Nach Untermischen von 99 g Stearinsäure wurde langsam abgekühlt; bei einem WAS-Gehalt von 69 % lag die Rührbarkeitsgrenze bei 105°C.

### Beispiel 21 (entspricht Beispiel 5 aus US 3 029 264)

Analog zu Beispiel 15 wurden 200 g Laurinsäure, 44,4 g Natriumisethionat, 100 g Ammoniumisethionat und 3,5 g Toluolsulfonsäure bei 120 - 125°C umgesetzt. Bereits bei einem WAS-Gehalt von 55 % (Epton-Titration) war das Gemisch nicht mehr rührbar. Nur durch eine Erhöhung der Reaktionstemperatur auf 180°C konnte die Kondensation fortgeführt werden. Das erhaltene, bei dieser Temperatur nicht mehr rührbare Produkt hatte einen WAS-Gehalt von 77 % (Epton-Titration) und einen Gehalt an freiem Hydroxyethansulfonat-Anion von 7,8 %.

Wie der Vergleich der beiden Beispiele 15 und 21 zeigt, erhält man auf die erfindungsgemäße Art und Weise, d.h. bei einem molaren Überschuß an Fettsäure, ein Tensidgemisch, dessen WAS-Gehalt an der Rührbarkeitsgrenze deutlich höher liegt als bei dem Produkt gemäß dem Stand der Technik. Außerdem konnte erfindungsgemäß der Gehalt an freiem Hydroxialkansulfonat deutlich abgesenkt werden.

## Patentansprüche

1. Tensidmischungen auf der Basis von Acyloxialkansulfonaten, hergestellt durch Umsetzung von mehr als einem Mol einer oder mehrerer Fettsäuren mit einem Mol eines Gemisches aus Alkali- und/oder Erdalkali-hydroxialkansulfonat und Ammonium-hydroxialkansulfonat in Gegenwart eines Veresterungskatalysators bei einer Temperatur von 100 bis 260°C.

2. Tensidmischungen nach Anspruch 1, dadurch gekennzeichnet, daß das Acyloxialkansulfonat ein Acylisethionat ist.

3. Tensidmischungen nach Anspruch 1, dadurch gekennzeichnet, daß das Alkali- und/oder Erdalkalihydroxialkansulfonat ein Natrium- und/oder Kaliumhydroxialkansulfonat ist.

4. Tensidmischungen nach Anspruch 1, dadurch gekennzeichnet, daß das Ammoniumkation des Hydroxialkansulfonats der Formel
R¹R²R³R⁴ N^{⊕}
entspricht, worin R¹, R², R³ und R⁴ Wasserstoff, C₁-C₄-Alkyl oder C₁-C₄-Hydroxyalkyl bedeuten.

5. Tensidmischungen nach Anspruch 1, hergestellt durch Umsetzung von Cocosfettsäure.

6. Tensidmischungen nach Anspruch 1, dadurch gekennzeichnet, daß das Acyloxialkansulfonat als gemischtes Natrium- und Ammoniumsalz vorliegt mit einem molaren Verhältnis von Na zu NH₄ von 98:2 bis 5:95.
